(19) Europäisches Patentamt
European Patent Office
Office européen des brevets

(11) Publication number: **0 077 105 B2**

(12) **NEW EUROPEAN PATENT SPECIFICATION**

(45) Date of publication of the new patent specification: **25.05.88**

(51) Int. Cl.[4]: **C 07 C 119/042,** C 08 G 18/73

(21) Application number: **82201256.3**

(22) Date of filing: **11.10.82**

(54) **Diisocyanate.**

(30) Priority: **10.10.81 NL 8104623**

(43) Date of publication of application: **20.04.83 Bulletin 83/16**

(45) Publication of the grant of the patent: **14.08.85 Bulletin 85/33**

(45) Mention of the opposition decision: **25.05.88 Bulletin 88/21**

(84) Designated Contracting States: **AT BE CH DE FR GB IT LI NL SE**

(56) References cited:
**DE-A-2 830 243**
**JP-A-44 004 542**
**US-A-3 311 654**

**Veba-Chemie AG, Produkt-Information 22-D-169-1-1 "Zwei neue Diisocyanate für lichtstabile Polyurethane".**

**The file contains technical information submitted after the application was filed and not included in this specification**

(73) Proprietor: **STAMICARBON B.V.**
**Mijnweg 1**
**NL-6167 AC Geleen (NL)**

(72) Inventor: **Van Geenen, Albert Arnold**
**Merkelbeekerstrasse 82**
**NL-6441 KM Brunssum (NL)**
Inventor: **Vincent, Joseph A. J. M.**
**Hubertusstrasse 72**
**NL-6191 PB Beek (NL)**

(74) Representative: **Hatzmann, Marinus Jan et al**
**OCTROOIBUREAU DSM P.O.Box 9**
**NL-6160 MA Geleen (NL)**

**EP 0 077 105 B2**

Courier Press, Leamington Spa, England.

**Description**

The invention relates to a group of novel diisocyanates, to intermediate products of these diisocyanates and to the use of these diisocyanates, or intermediate products thereof, for the preparation of polyurethanes.

From US-patent specification No. 3,311,654 various classes of aliphatic diisocyanates are known, as well as a method for the preparation thereof.

The German patent application No. 2830243 describes aliphatic diisocyanates containing only primary isocyanate groups.

Multifunctional isocyanates are frequently used in the preparation of polyurethanes. Examples of these kinds of uses are the preparation of polyurethane foam, lacquers, and elastomers, in which the elastomeric polyurethanes can, moreover, be divided into solid polyurethanes and microcellular polyurethane foams; or moulded objects.

Commercially available aromatic diisocyanates include toluenediisocyanate (TDI), polymethylpolyphenylisocyanate and methyldiphenylisocyanate.

In addition, a number of isocyanates is available which do not contain the combination of an isocyanate group with an aromatic nucleus, such as hexane(1,6)diisocyanate (HMDI), isophoronediisocyanate (IPDI) and trimethylhexane(1,6) diisocyanate (TMDI), which consists of a mixture of 2,2,4- and 2,4,4-isomers.

In connection with the toxicity, volatility and reactivity of the various diisocyanates, it is often preferred not to use the diisocyanate itself, but a reaction product having better properties in this respect.

This particularly concerns the adduct of the diisocyanate with a polyol (e.g. a diol or a triol), the dimer or the trimer of the diisocyanate, the trimer advantageously possessing an isocyanurate structure, and the reaction product of two or more diisocyanates with water.

In many cases a blocked polyisocyanate is used; particularly in so-called one-component systems. The most important commercially used blocking agents are phenols, oximes and lactams.

The object of the invention is to provide a diisocyanate, and particularly a diisocyanate based on a branched or unbranched aliphatic compound, which can be used in a simple manner, without many disturbing side reactions, for the production of reaction products suitable for further processing.

The diisocyanate according to the invention is characterized by formula

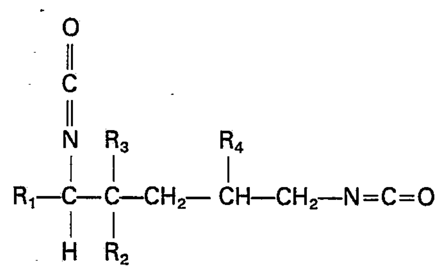

where $R_1$ represents an alkyl group with 1—10 C atoms, a phenyl group, or a group according to formula

$$R_1''—O—R_1'—$$

in which $R_1'$ and $R_1''$ represent alkyl groups with 1—10 C atoms, and where $R_2$, $R_3$ and $R_4$ are the same or different and are chosen from the group consisting of

— alkyl group with 1—10 C atoms,

— alkoxy group with 1—10 C atoms,

— —H

According to a preferred embodiment of the invention, $R_1$ is an alkyl group, more specifically a methyl group, and $R_{2-4}$ are hydrogen atoms. The compound thus obtained is 1,5 hexanediisocyanate.

The great advantage of the diisocyanate according to the invention is that it can be reacted selectively via one of the two isocyanate groups with, for instance, a polyol to form a polyisocyanate. It is quite possible to react three diisocyanate molecules with a conventional triol to form a high-molecular triisocyanate.

With these isocyanates, unlike HMDI or TDMI, there will be no or relatively few undesired side reactions resulting in a partial loss of the functionality of the isocyanate owing to the fact that the reaction cannot be performed with the required selectivity.

In the oligomerisation of the diisocyanate, and especially the trimerisation to isocyanurate, the same advantages occur. Furthermore, it has been found, that the oligomerisation proceeds very easily to high yields of oligomer i.e. to a product having a very low free diisocyanate content, without the formation of gels.

It is possible to prepare an oligomer of the well-known 1,6-hexane-diisocyanate, by oligomerising to relatively low conversions and subsequent removing of the unreacted diisocyanate. It is however not

possible to react this diisocyanate directly and in high yields to a substantially pure oligomer as this results in extensive gel-building.

An alternative for the oligomerisation is the reaction of two or more diisocyanates with each other under the influence of water. As this is an equilibrium reaction, the free diisocyanate content increases with time.

With the diisocyanate according to the invention it is possible to produce an oligomer having a very low free diisocyanate content, which does not increase with time.

The invention, therefore, also relates to an adduct, having one or more terminal isocyanate groups, of the diisocyanate with a compound containing one or more active hydrogen atoms, such as di- or polyols, di- or polyamines, and water.

The diisocyanate can be prepared as known in the art for analogous compounds, for instance from the corresponding diamine compound by treating it with phosgene or other known reagents, such as diphenylcarbonate.

These preparation processes are known to the man skilled in the art and need not be further elucidated here.

The diamine can be prepared from, amongst others, the corresponding delta ketonitrile, for example as described in Chem. Ber. *99* (10) p. 3385—87 (1966).

Generally, the uses of diisocyanates are known to the man skilled in the art. In literature many recipes are described. In this connection see, for instance, also the European patent applications 4115 and 4116 laid open to public inspection.

The diisocyanates according to the invention can advantageously be used for single and two-component lacquers. As in the case of elastomers, the diisocyanate or a reaction product thereof, such as an adduct or a prepolymer, is reacted with one or more diols and/or polyols.

Usually, the diisocyanates or reaction products thereof are reacted with one or more polyols to urethanes. Use can be made of polymeric polyols. Such polymeric polyols may contain from 2 to 5, preferably 2, hydroxyl groups per molecule. Suitable polymeric polyols include the polyoxyalkylene glycols containing $C_{2-4}$ oxyalkylene units e.g. polyethylene glycol, polypropylene glycol, random or block copolymers of ethylene oxide and propylene oxide, adducts of ethylene oxide or propylene oxide to a trihydroxy compound, polyactone diols derived from a lactone containing 5—12 atoms, for instance hydroxyl-terminated polycaprolactone or polyvalerolactone or co-polyactones. Hydroxylterminated polyesters, butadiene polymers containing hydroxyl groups or copolymers of butadiene with e.g. acrylonitrile, isoprene and/or styrene containing hydroxyl groups, and hydroxyl group containing acrylates. The polyesters have usually been obtained by reacting one or more $C_4$—$C_{10}$ aliphatic and/or aromatic carboxylic acids, for instance adipic acid, azelaic acid, phthalic acid or terephthalic acid, and one or more aliphatic and/or cycloaliphatic diols, for instance ethylene glycol, diethylene glycol, 1,4-butane diol, 1,6-hexane diol, propylene glycol or 1,4-(hydroxymethyl)-cyclohexane.

The butadiene (co)polymers containing hydroxyl groups usually contain more than 2, e.g. between 2.0 and 2.3, hydroxyl groups. Preferably use is made of a polymeric polyol having a molecular weight between 500 and 2000. Particularly suitable are the polyalkylene glycols, the hydroxyl-terminated polylactones, the hydroxyl-terminated polyesters and the hydroxyl group containing polyacrylates.

In some applications also use is made of a low molecular-weight diol that can function as chain extenter in addition to the polymeric polyol, such diols having a molecular weight of between 60 and 200, as hereinbefore set forth. Examples are aliphatic unbranched diols e.g. ethylene glycol, 1,4-butane diol, and 1,6-hexane diol; branched diols e.g. propylene glycol and 2,2-dimethyl-1,4-butane diol; low molecular-weight polyalkylene glycols e.g. diethylene glycol, triethylene glycol and cycloaliphatic diols e.g. 1,4(hydroxymethyl)-cyclohexane. Preferably use is made of $C_2$—$C_6$ aliphatic diols.

If the said low molecular-weight diols are used they are present in an amount so that up to 20, preferably from 0.5 to 5.0, and particularly from 1.0 to 4.0, hydroxyl groups are present in the starting materials deriving from the said low molecular-weight diol, for every hydroxyl group deriving from the polymeric polyol(s).

The diisocyanates according to the present invention are particularly suitable for the preparation of lacquers, as they do not have the tendency to yellow after exposure to UV-light.

These lacquers include the one- and two-component systems. In the case of a one-component system all components of the lacquer are included in the composition. Reaction of the isocyanate is however, prevented by blocking (or protecting) the reactive isocyanate group with a conventional blocking agent. These blocking agents include i.e. phenols, oximes and lactams. Upon heating the isocyanate group is feed and reacts with the hydroxy compounds to a polyurethane coating.

In the two-component systems, the isocyanate-component and the hydroxy-component of the lacquer are mixed shortly before the use thereof. To prevent undue losses of these prepared lacquer composition prior to the use thereof, it is essential that it can be used up to some hours after the preparation. It is one of the surprising effects of the present invention that the so-called 'pot-life' of lacquers based on these diisocyanate or reaction products thereof is much longer than the 'pot-life' of lacquers based upon the closely related 1,6 hexane diisocyanate.

In addition to the diisocyanate and the hydroxy components, these lacquers may contain usual other additives such as stablisers, pigments, catalysts, solvents etc.

Specific applications of lacquers based on the diisocyanates according to the present invention are industrial wood lacquers, airplane-lacquers, textile lacquers, car-repair lacquers and leather lacquers.

Example I

212 g 1,5 hexanediamine was dissolved in 28 ml methanol. After the solution had been cooled down to 10°C, 34 ml concentrated hydrochloric acid (s.g. 1.19) was added in drops, in which process care was taken not to allow the temperature of the solution to rise above 30°C.

After removal of the solvents by evaporation, the product was dried in a vacuum drying oven till it was completely free of water.

34 g of the resulting amine salt was then suspended, in the form of a very fine powder, in 200 ml Decalin (decahydronaphthalene). After raising of the temperature to 180°C phosgene was introduced below the surface of the liquid during very intensive stirring. After about 1/3 mole phosgene per hour had been passed through for 10 hours, the liquid was cooled down. The liquid was filtered, and the filtrate was distilled at 15 mm Hg.

After removal of the Decalin, a clear liquid was obtained with a boiling point of 120°C (15 mm Hg).

The IR spectrum of this liquid shows that the compound obtained contains $—CH_3$, $—CH_2$ and —OCN groups, which is indicative of the fact that 1,5 hexanediisocyanate was obtained.

In this connection it is noted that it is not essential to start from the hydrochloric acid salt of the amine. Particularly on a larger scale it is unnecessary. On a laboratory scale, however, it makes the synthesis simpler.

Example II

71 g 1,5 diaminohexane, dissolved in 1 litre of O-dichlorobenzene, was heated to 110°C. At this temperature $CO_2$-gas was passed over the stirred solution, until no more uptake of $CO_2$ occurred. Thereafter the temperature was decreased to 95°C with continuing $CO_2$-flow. This was continued for 4 hours.

After cooling to −10°C of the thus obtained carbamate suspension, 150 g phosgene was fed to the suspension with a flow of 100 g/h. Thereafter the phosgene-flow was reduced to 10 g/h. Simultaneously the mixture was heated with about 5°C/5 min. to 45°C. Heating was continued with 5°C/10 min. until 70°C.

The phosgene flow was then increased to 30 g/h and the mixture was heated with 5°C/5 min. until a temperature of 130°C was obtained. This situation was continued for 16 h.

Unreacted phosgene was removed from the system by flushing with nitrogen, whereafter the mixture was cooled to ambient temperature. After filtration of the mixture the filtrate obtained was distilled at 15 mm Hg. The fraction obtained at 123°C consisted of 70 g of 1,5 hexane diisocyanate.

Example III

The 1,5 hexanediisocyanate prepared according to Example II was oligomerized using 0,8 wt.% DABCO-TMR (RTM of Air Products and Chemicals) as catalyst. The resulting (52 wt.%) solution of oligomer in butylacetate/acetonitrile (1/1 by volume), having an NCO-content of 19 wt.% based on ologomer and a free diisocyanate content of 0.15 wt.%, was used in a hydroxyacrylate, two-component lacquer (Lacquer I).

This lacquer was compared with the same lacquer composition based upon Desmodur N (RTM of Bayer AG), which is a reaction product of 1,6 hexanediisocyanate (Lacquer II).

A. Potlife

The viscosity of the lacquers was measured (in sec. with a FORD 4 cup) after certain time intervals after mixing the two components.

TABLE 1

| Time | Lacquer* I | Lacquer** II |
|---|---|---|
| 8 h | 18.6 | 19.0 |
| 48h | 20.8 | 22.2 |
| 120 h | 25.2 | 39.2 |
| Gel after | 288 h | 144 h |

* According to the invention
** Not according to the invention

This results in a pot life which is twice as long for the lacquer based upon the diisocyanate according to the invention.

B. Chemical resistance

1. The same lacquers as used under A were applied to a glass plate. The resistance against the action of acetone for 1 minute was tested after various times.

TABLE 2

| Time | Lacquer | Lacquer II |
|---|---|---|
| 8 h | 1 | 1 |
| 24 h | 3 | 2 |
| 120 h | $3\frac{1}{2}$ | 3 |

The scale used was:

1. very bad
2. bad
3. moderate
4. good
5. perfect.

2. The same lacquers were applied to mahogany veneered chipboard. After 7 days, the resistance against various treatments was measured.

| | Lacquer I | Lacquer II |
|---|---|---|
| demineralised water (24 h) | 5 | 5 |
| ethanol/water (16 h) | $5^{\times}$ | $5^{-}$ |
| Coffee (16 h) | 5 | 5 |
| Lipstick (16 h) | 5 | 5 |
| Acetic acid (10%) (16 h) | 5 | 5 |
| Acetone (1 min.) | 4 | 3 |

From the above results can be concluded that the resistance of the lacquers based upon the diisocyanates according to the invention is at least as good or even better than of those based upon the known diisocyanates.

**Claims**

1. Diisocyanate characterized by formula I:

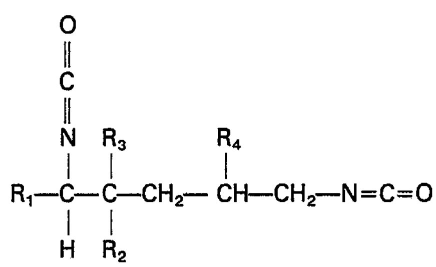

(I)

where $R_1$ represents an alkyl group with 1—10 C atoms, a phenyl group, or a group according to formula

$$R_1''—O—R_1'—$$

in which $R_1'$ and $R_1''$ represent alkyl groups with 1—10 C atoms, and where $R_2$, $R_3$ and $R_4$ are the same or different and are chosen from the group consisting of

— alkyl group with 1—10 C atoms,
— alkoxy group with 1—10 C atoms,
— H.

2. Diisocyanate according to claim 1, characterized in that $R_1$ represents an alkyl group and $R_{2-4}$ represent a hydrogen atom.

3. Diisocyanate according to claim 1 or 2, characterized in that $R_1$ is a methyl group.

4. Adduct having one or more terminal isocyanate groups of a diisocyanate according to any one of claims 1—3 with a compound containing one or more active hydrogen atoms, other than said diisocyanate.

5. Adduct according to claim 4, characterized in that a polyol or a triol is used.

6. Application of a diisocyanate, or a diisocyanate adduct according to any one of claims 1—5 for the preparation of a polyurethane.

7. Application according to claim 6 for the preparation of a polyurethane coating, or a lacquer or a polyurethane elastomer.

8. Moulded object consisting wholly or partly of a polyurethane obtained while applying the application according to claim 6 or 7.

**Patentansprüche**

1. Diisocyanat, gekennzeichnet durch die Formel

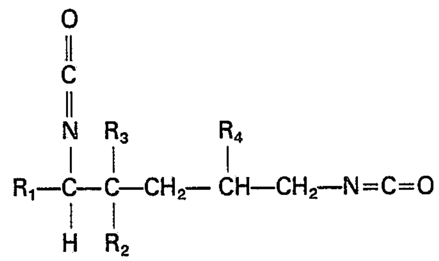

(I)

worin $R_1$ eine Alkylgruppe mit 1—10 C-Atomen, eine Phenylgruppe oder eine Gruppe der Formel

$$R_1''—O—R_1'—$$

darstellt, in welcher $R_1'$ und $R_1''$ Alkylgruppen mit 1—10 C-Atomen darstellen, und worin $R_2$, $R_3$ und $R_4$ gleich oder verschieden sind und ausgewählt sind aus der Gruppe bestehend aus:
— Alkylgruppe mit 1—10 C-Atomen,
— Alkoxygruppe mit 1—10 C-Atomen,
— H.

2. Diisocyanat nach Anspruch 1, dadurch gekennzeichnet daß $R_1$ eine Alkylgruppe darstellt und $R_{2-4}$ ein Wasserstoffatom darstellen.

3. Diisocyanat nach Anspruch 1 oder 2, dadurch gekennzeichnet, daß $R_1$ eine Methylgruppe ist.

4. Addukt mit einer oder mehreren endständigen Isocyanat-gruppen eines Diisocyanats gemäß einem der Ansprüche 1—3 mit einer anderen Verbindung als dieses Diisocyanat, welche ein oder mehrere aktive Wasserstoffatome enthält.

5. Addukt nach Anspruch 4, dadurch gekennzeichnet, daß ein Polyol oder ein Triol verwendet wird.

6. Verwendung eines Diisocyanats oder eines Diisocyanat-addukts gemäß einem der Ansprüche 1—5 für die Herstellung eines Polyurethans.

7. Verwending nach Anspruch 6 für die Herstellung einer Polyurethanbeschichtung, oder eines Lackes oder eines Polyurethanelastomers.

8. Geformter Gegenstand bestehend ganz oder zum Teil aus einem Polyurethan, das bei der Verwendung gemäß Anspruch 6 oder 7 erhalten wurde.

**Revendications**

1. Diisocyanate, caractérisé par la formule

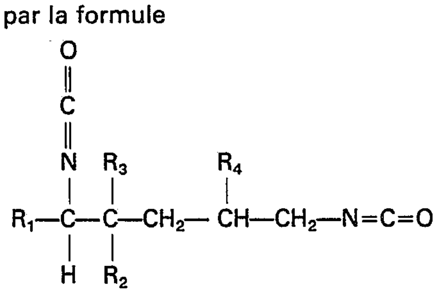

dans laquelle $R_1$ représente un radical alkyle de 1 à 10 atomes de C, un radical phényle ou un groupe de formule

$$R_1''—O—R_1'—$$

dans laquelle $R_1'$ et $R_1''$ représentent des radicaux alkyle de 1 à 10 atomes de C, et dans laquelle $R_2$, $R_3$ et $R_4$ qui peuvent être identiques ou différents, sont chacun choisis dans le groupe comprenant

— un radical alkyle de 1 à 10 atomes de C,
— un radical alcoxy de 1 à 10 atomes de C,
— H.

2. Diisocyanate selon la revendication 1, caractérisé en ce que $R_1$ représente un radical alkyle et $R_{2-4}$ représentent chacun un atome d'hydrogène.

3. Diisocyanate selon la revendication 1 ou 2, caractérisé en ce que $R_1$ est un radical méthyle.

4. Produit d'addition comportant un ou plusieurs groupes isocyanates terminaux d'un diisocyanate selon l'une quelconque des revendications 1 à 3, avec un composé contenant un ou plusieurs atomes d'hydrogène actif, autre que ledit diisocyanate.

5. Produit d'addition selon la revendication 4, caractérisé en ce qu'on utilise un polyol ou un triol.

6. Application d'un diisocyanate ou d'un produit d'addition de diisocyanate selon l'une quelconque des revendications 1 à 5 pour la préparation d'un polyuréthane.

7. Application selon la revendication 6 pour la préparation d'un revêtement de polyuréthane, ou d'un vernis ou d'un élastomère de polyuréthane.

8. Objet moulé consistant en totalité ou en partie d'un polyuréthane obtenu pendant qu'on effectue l'application selon la revendication 6 ou 7.